# EUROPEAN PATENT SPECIFICATION

(11) **EP 4 285 970 B1**
(45) Date of publication and mention of the grant of the patent: **28.08.2024**
(21) Application number: 22176697.5
(22) Date of filing: 01.06.2022
(51) Int. Cl.: A61M 11/00, A61M 15/00

(54) **QUICK-RELEASE NEBULIZER**
SCHNELL ABNEHMBARER ZERSTÄUBER
NÉBULISEUR À LIBÉRATION RAPIDE

(43) Date of publication of application: 06.12.2023
(73) Proprietor: GaleMed Corporation, Yilan County 268 (TW)
(72) Inventor: CHEN, Po-Chang, 268 Yilan County (TW); WANG, Hsin-Chen, 268 Yilan County (TW); YANG, Chia-Chin, 268 Yilan County (TW); CHEN, Hao-Hsiang, 268 Yilan County (TW); HSU, Chun-Wei, 268 Yilan County (TW)
(74) Representative: 2K Patentanwälte Blasberg Kewitz & Reichel

(56) References cited:
- WO-A1-2017/195632
- CN-A- 111 956 917
- TW-B- I 761 074
- US-A1- 2016 361 506
- US-A1- 2018 361 084

## Description

### BACKGROUND

### Technical Field

The disclosure relates to a nebulizer structure, particularly to a quick-release nebulizer.

### Description of Related Art

A related-art nebulizer usually uses compressed gas supplied by a gas supply to nebulize an agent, and then the nebulized agent is driven by the compressed gas to be ejected for being sucked by a patient so that the nebulized agent can be diffused to the patient's lung though his or her bronchus.

However, after such a nebulizer has been used, its spraying head must be removed and washed to prevent contagious germs from spreading through the respiratory organs. Thus, how to conveniently and rapidly install and remove a spraying head becomes an issue to be researched. US2018/61084A1 discloses a nebulizer with a press-type medicine cup. The nebulizer includes a body, a medicine cup, an engagement structure, and a multi-directional resilient member. The engagement structure includes an inner protruding base and an outer protruding block disposed outside the inner protruding base. The inner protruding base and the outer protruding block extend from the body and the medicine cup. The inner protruding base includes a cavity inside and includes two through holes. The outer protruding block includes two engagement grooves. The multi-directional resilient member is installed in the cavity and includes a press element, two straight resilient arms extend from the press element, two oblique resilient arms are bent from the two straight resilient arms, and each oblique resilient arm has a block member. The two block members are inserted through the two through holes to be engaged with the two engagement grooves.

CN111956917B disclose a further nebuliser with a detachable head. These two documents do not disclose at least a T-shaped block received in T-shaped trench.

In view of this, the inventors have devoted themselves to the above-mentioned related art, researched intensively and cooperated with the application of science to try to solve the above-mentioned problems. Finally, the invention which is reasonable and effective to overcome the above drawbacks is provided.

### SUMMARY

An object of the disclosure is to provide a quick-release nebulizer, which utilizes the spraying seat being assembled to or disassembled from the body along the radial direction and the movable member being pushed along the axial direction to make the engaging block be embedded in or separated from the engaging trench so as to rapidly install or remove the spraying head.

In an embodiment of the disclosure, the disclosure provides a quick-release nebulizer, which includes a main body, a spraying head, and an engagement structure. The main body includes a body. The body defines an axial direction and a radial direction. The spraying head includes a spraying seat assembled to or disassembled from the body along the radial direction. The engagement structure includes a notch formed on one of the body and the spraying seat, a T-shaped trench formed on an inner wall of the notch along the axial direction, an engaging trench formed on another one of the body and the spraying seat and arranged corresponding to the notch, and a movable member received in the notch. A side of the movable member is extended with a handle exposed from the notch, another side thereof is extended with a T-shaped block slidably received in the T-shaped trench, and still another side thereof is protruded with an engaging block embedded in or separated from the engaging trench.

Accordingly, the spraying seat is assembled to the body through the sliding rail structure along the radial direction, and then the handle is pushed upward along the axial direction to drive the whole movable member to move upward to make the engaging block be embedded in the engaging trench to lock the spraying seat. As a result, the spraying head may be rapidly assembled to the main body. On the contrary, the handle is pushed downward along the axial direction to drive the whole movable member to move downward to make the engaging block disengage from the engaging trench to release the spraying seat, and then the spraying seat is removed from the body through the sliding rail structure along the radial direction. As a result, the spraying head may be rapidly disassembled to the main body. Therefore, the quick-release nebulizer has the function of conveniently and rapidly assembling and disassembling the spraying head.

### BRIEF DESCRIPTION OF THE DRAWINGS

FIG. 1 is an exploded view of the quick-release nebulizer of the disclosure;
FIG. 2 is another exploded view of the quick-release nebulizer of the disclosure;
FIG. 3 is still another exploded view of the quick-release nebulizer of the disclosure;
FIG. 4 is an exploded cross-sectional view of the quick-release nebulizer of the disclosure;
FIG. 5 is the first assembled cross-sectional view of the quick-release nebulizer of the disclosure;
FIG. 6 is the second assembled cross-sectional view of the quick-release nebulizer of the disclosure;
FIG. 7 is the third assembled cross-sectional view of the quick-release nebulizer of the disclosure;
FIG. 8 is the fourth assembled cross-sectional view of the quick-release nebulizer of the disclosure;
FIG. 9 is the fifth assembled cross-sectional view of the quick-release nebulizer of the disclosure; and
FIG. 10 is a perspective assembled view of the quick-release nebulizer of the disclosure.

### DETAILED DESCRIPTION

The technical contents of this disclosure will become apparent with the detailed description of embodiments accompanied with the illustration of related drawings as follows. It is intended that the embodiments and drawings disclosed herein are to be considered illustrative rather than restrictive.

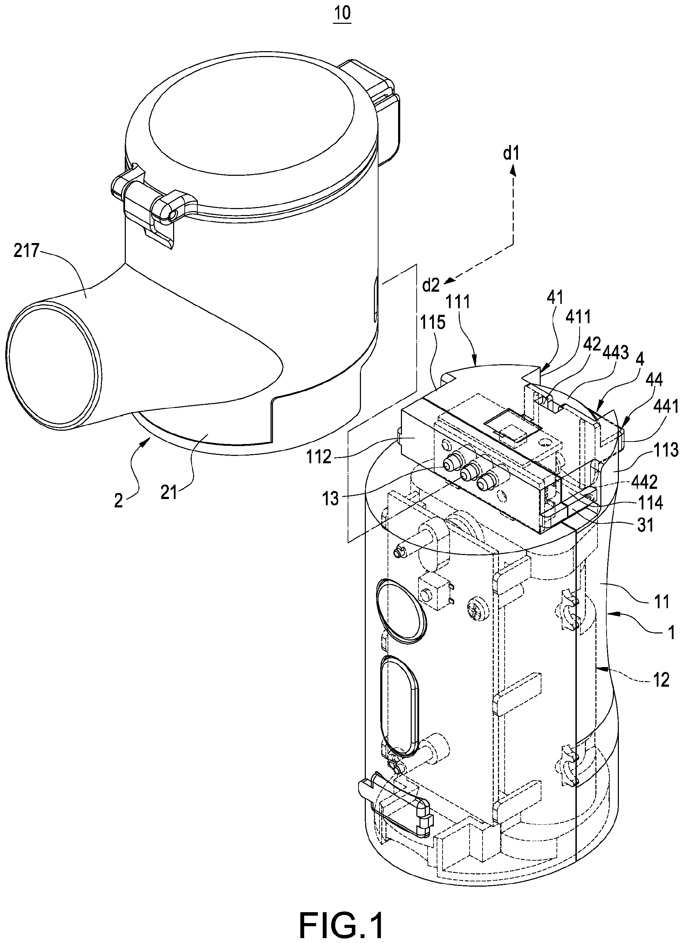

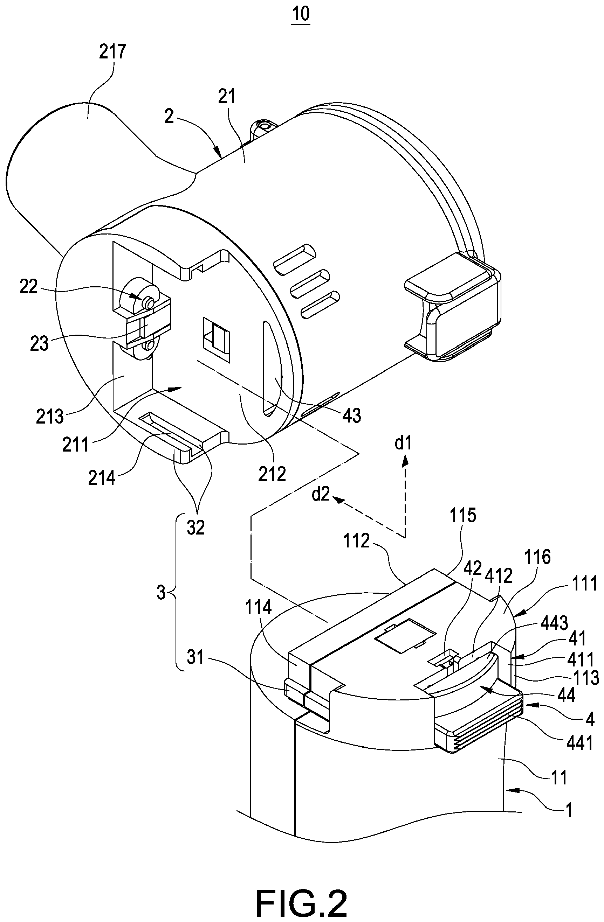

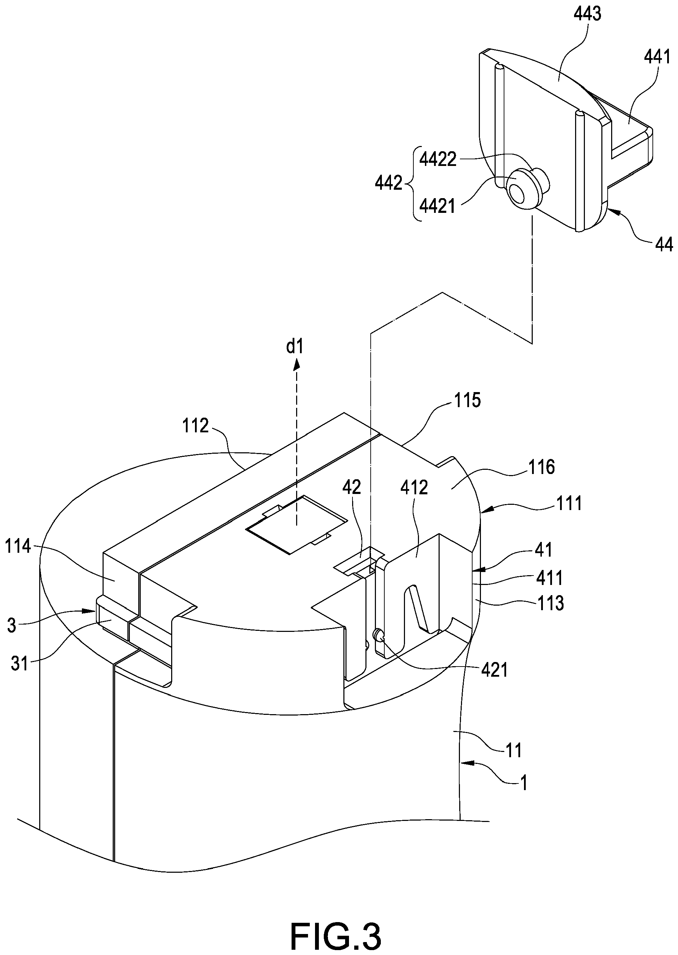

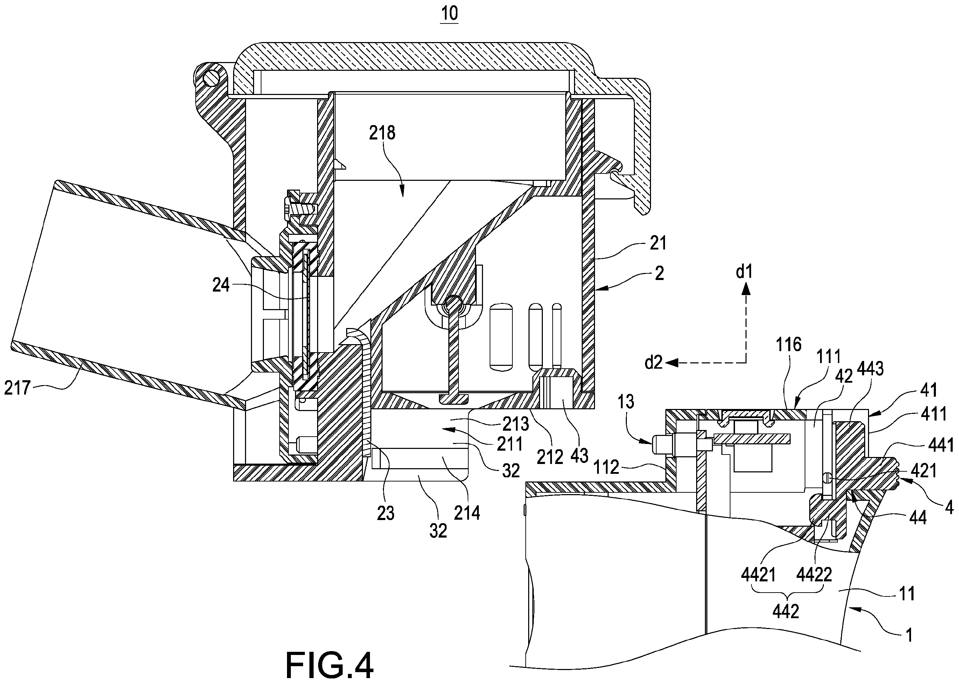

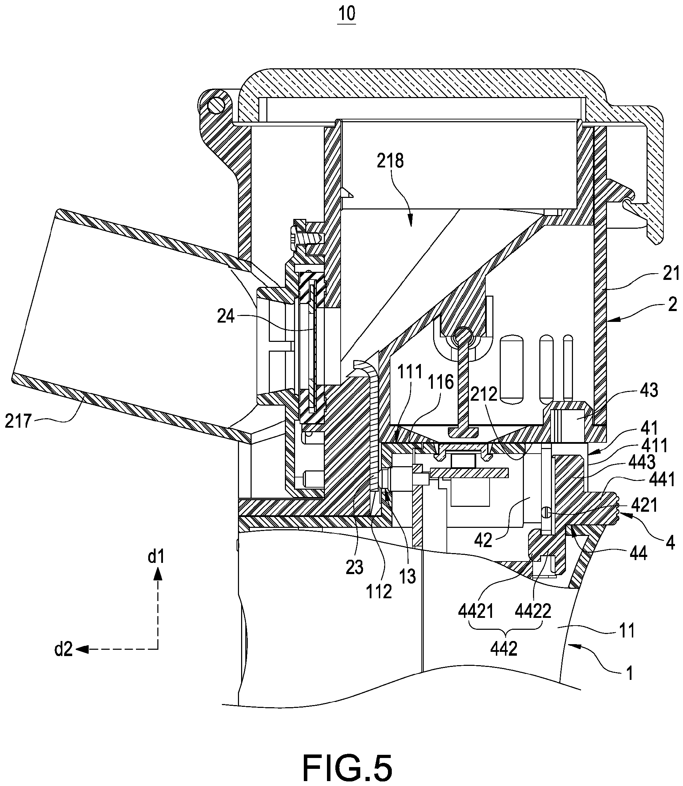

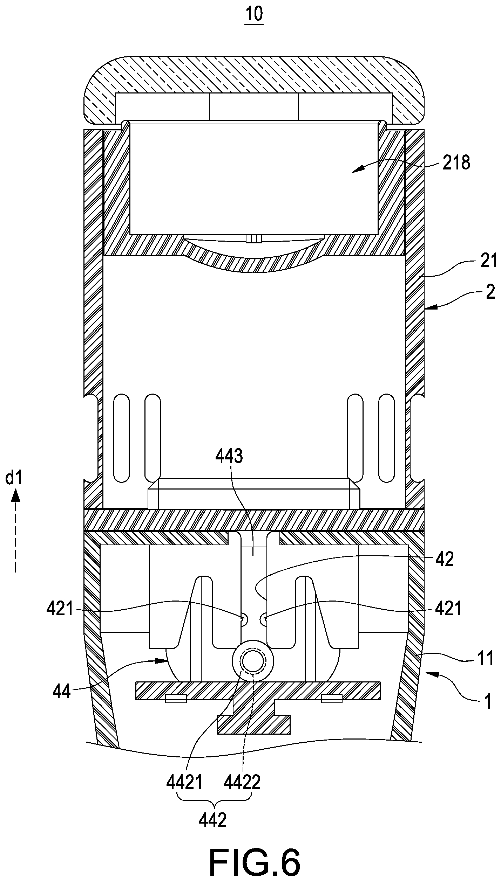

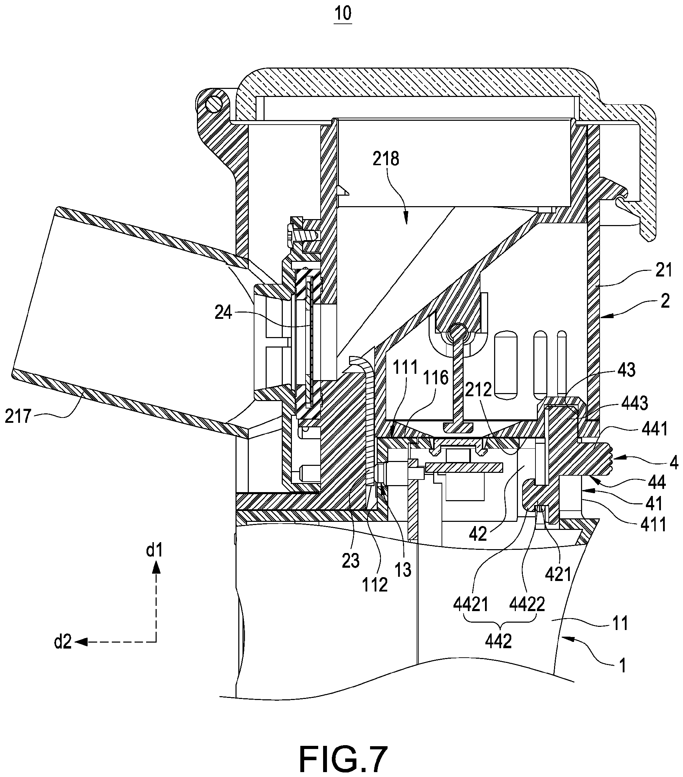

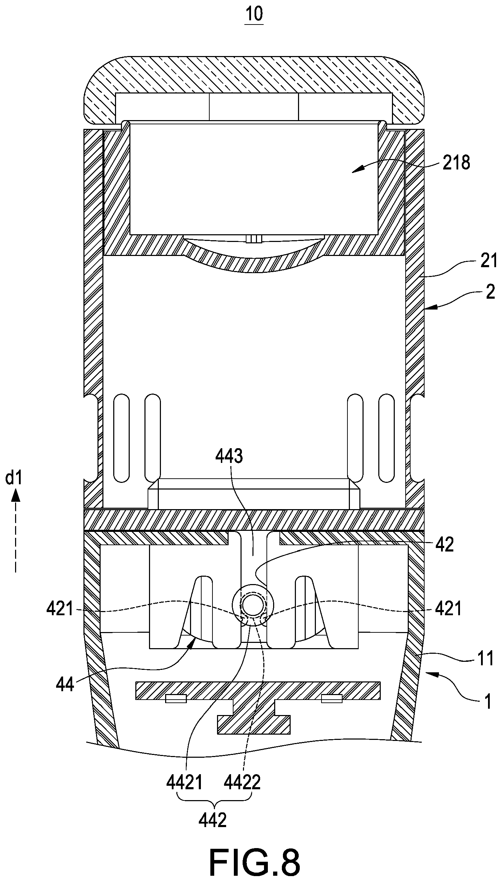

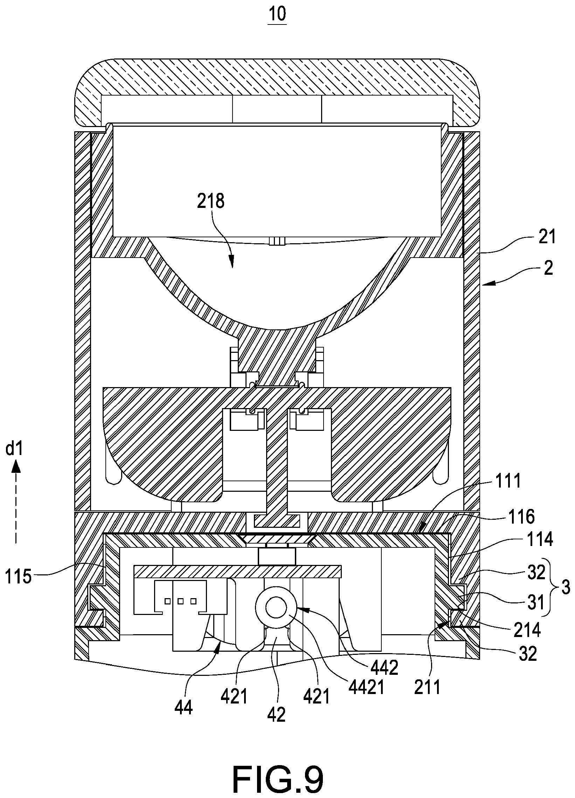

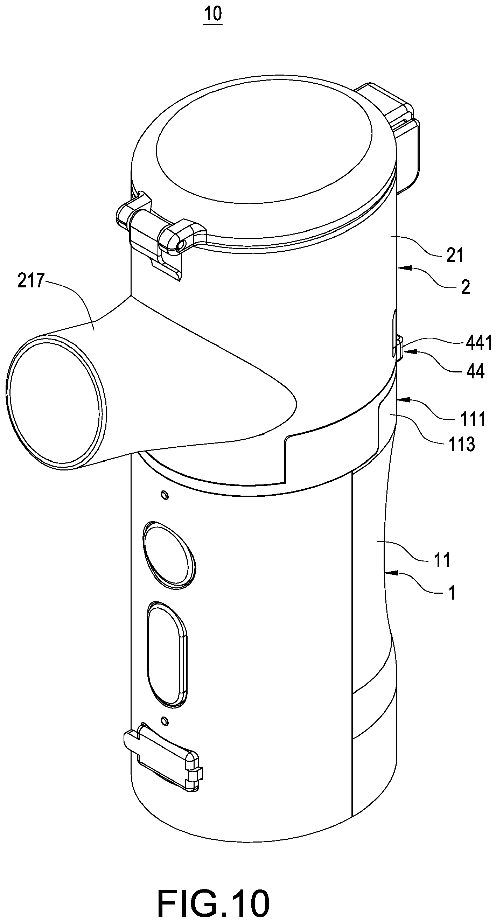

Please refer to FIGS 1-10. The disclosure provides a quick-release nebulizer. The nebulizer 10 includes a main body 1, a spraying head 2 and an engagement structure 4.

As shown in FIGS. 1-10, the main body 1 includes a body 11, a power assembly 12 received in the body 11 and a first conductive member 13 electrically connected to the power assembly 12. The body 11 defines an axial direction d1 and a radial direction d2. In the embodiment, the power assembly 12 includes a circuit board and a battery, and the first conductive member 13 includes multiple pogo pins, but not limited to these.

In addition, the top of the body 11 is protruded with a block 111. The block 111 has a front side 112, a rear side 113, a left side 114, a right side 115 and a top side 116. The first conductive member 13 is installed on the front side 112.

As shown in FIGS. 1, 2 and 4-10, the spraying head 2 includes a spraying seat 21 assembled to (assemblable with) or disassembled from (detachable with) the body 11 along the radial direction d2. The bottom and the outer periphery of the spraying seat 21 are formed with a recess 211. The recess 211 has an inner bottom 212 and a U-shaped inner sidewall 213 disposed on a side of the inner bottom 212.

In detail, as shown in FIGS 1-3 and 9, the quick-release nebulizer 10 of the disclosure further includes a sliding rail structure 3. The sliding rail structure 3 includes one or more sliders 31 formed on either one of the body 11 and the spraying seat 21 and one or multiple pairs of rails 32 formed on the other one of the body 11 and the spraying seat 21.

In the embodiment, the sliders 31 are two in number, and the rails 32 are two pairs in number, but not limited to these. The two sliders 31 are formed by protruding from the left side 114 and the right side 115 of the block 111. Two sides of the U-shaped inner sidewall 213 are formed with two rail grooves 214 along the radial direction d2. The U-shaped inner sidewall 213 is formed with each pair of rails 32 corresponding to the upper side and the lower side of each rail groove 214 so that each pair of rails 32 is arranged along the radial direction d2 and in a top-and-bottom configuration. Each slider 31 is slidable and stopped between each pair of rails 32 so as to make the spraying seat 21 be able to be assembled to or disassembled from the body 11 along the radial direction d2.

In addition, the spraying head 2 further includes a second conductive member 22 and a no-water detection probe 23. The second conductive member 22 is installed on the U-shaped inner sidewall 213 and abuts against the first conductive member 13. The power assembly 12 transmits electricity to the no-water detection probe 23 only via the first conductive member 13. The second conductive member 22 in the embodiment includes, but not limited to, multiple conductive terminals.

Also, the outside of the spraying seat 21 is extended with a nozzle 217 and the inside thereof is disposed with an agent chamber 218 communicating with the nozzle 217. The spraying head 2 further includes a nebulizing sheet 24 installed at the junction between the nozzle 217 and the agent chamber 218. An end of the no-water detection probe 23 stretches in the agent chamber 218 and is arranged corresponding to the nebulizing sheet 24. The nebulizing sheet 24 is electrically connected with the second conductive member 22. The power assembly 12 transmits electricity to the nebulizing sheet 24 via the first conductive member 13 and the second conductive member 22. The no-water detection probe 23 is used to detect if liquid exists after being electrified. The nebulizing sheet 24 vibrates after being electrified to nebulize liquid in the agent chamber 218 and allows the nebulized liquid to pass through for being ejected through the nozzle 217.

As shown in FIGS. 1-10, the engagement structure 4 includes a notch 41 formed on one of the body 11 and the spraying seat 21, a T-shaped trench 42 formed on an inner wall of the notch 41 along the axial direction d1, an engaging trench 43 formed on the other one of the body 11 and the spraying seat 21 and arranged corresponding to the notch 41, and a movable member 44 movably received in the notch 41.

In addition, a side of the movable member 44 is extended with a handle 441 exposed from the notch 41, another side thereof is extended with a T-shaped block 442 slidably received in the T-shaped trench 42, and still another side thereof is protruded with an engaging block 443 along the axial direction d1, which may be embedded in or separated from the engaging trench 43.

Further, the T-shaped block 442 has a head portion 4421 and a rod portion 4422 connected between the head portion 4421 and the movable member 44. The inner wall of the T-shaped trench 42 is extended with two resistant bumps 421 that are frictional with the rod portion 4422.

Also, the notch 41 of the embodiment is formed by indenting the rear side 113 and the top side 116. The notch 41 has an opening 411 formed on the rear side 113 and an inner end face 412 corresponding to the opening 411. The T-shaped trench 42 is formed by indenting the inner end face 412 and the top side 116. The engaging trench 43 is formed by indenting the inner bottom 212. The handle 441 is exposed from the opening 411.

In detail, when the engaging block 443 is embedded in the engaging trench 43, the rod portion 4422 goes up over the two resistant bumps 421 and rubs the two resistant bumps 421 to transmit a hand-operated resistance. After that, the two resistant bumps 421 abut against the bottom edge of the rod portion 4422 to temporarily lock the position of the movable member 44 until a user downward pushes the handle 441 to move the rod portion 4422 to go down over the two resistant bumps 421 so as to release the position of the movable member 44 to make the engaging block 443 smoothly disengage from the engaging trench 43.

As shown in FIGS. 1-10, when using the quick-release nebulizer 10 of the disclosure, the spraying seat 21 is assembled to the body 11 through the sliding rail structure 3 along the radial direction d2 first, and then the handle 441 is pushed upward along the axial direction d1 to drive the whole movable member 44 to move upward so as to make the engaging block 443 be embedded in the engaging trench 43 to lock the spraying seat 21. As a result, the spraying head 2 may be rapidly assembled to the main body 1. On the contrary, the handle 441 is pushed downward along the axial direction d1 to drive the whole movable member 44 to move downward so as to make the engaging block 443 disengage from the engaging trench 43 to release the spraying seat 21, and then the spraying seat 21 is removed from the body 11 through the sliding rail structure 3 along the radial direction d2. As a result, the spraying head 2 may be rapidly disassembled from the main body 1. Therefore, the quick-release nebulizer 10 has the function of conveniently and rapidly assembling and disassembling the spraying head 2.

## Claims

1. A nebulizer comprising:
a main body (1), comprising a body (11), and the body (11) defining an axial direction (d1) and a radial direction (d2);
a spraying head (2), comprising a spraying seat (21) assembled to or disassembled from the body (11) along the radial direction (d2); and
an engagement structure (4), comprising a notch (41) disposed on one of the body (11) and the spraying seat (21), a T-shaped trench (42) disposed on an inner wall of the notch (41) along the axial direction (d1), an engaging trench (43) disposed on another one of the body (11) and the spraying seat (21) and arranged corresponding to the notch (41), and a movable member (44) received in the notch (41), wherein a side of the movable member (44) is extended with a handle (441) exposed from the notch (41), another side of the movable member (44) is extended with a T-shaped block (442) slidably received in the T-shaped trench (42), and still another side of the movable member (44) is protruded with an engaging block (443) embedded in or separated from the engaging trench (43) along the axial direction.

2. The nebulizer of claim 1, wherein the T-shaped block (442) comprises a head portion (4421) and a rod portion (4422) connected between the head portion (4421) and the movable member (44), and an inner wall of the T-shaped trench (42) is extended with two resistant bumps (421) being frictional with the rod portion (4422).

3. The nebulizer of claim 2, further comprising a sliding rail structure (3), wherein the sliding rail structure (3) comprises at least one slider (31) disposed on either one of the body (11) and the spraying seat (21) and at least one pair of rails (32) disposed on another one of the body (11) and the spraying seat (21), the at least one pair of rails (32) is arranged along the radial direction (d2) and in a top-and-bottom configuration, and the at least one slider (31) is slidable and stopped between the at least one pair of rails (32).

4. The nebulizer of claim 3, wherein a top of the body (11) is protruded with a block (111), the block (111) comprises a rear side (113), a left side (114), a right side (115) and a top side (116), the slider (31) is two in number, the two sliders (31) protrude from the left side (114) and the right side (115), and the notch (41) is indented on the rear side (113) and the top side (116).

5. The nebulizer of claim 4, wherein the notch (41) comprises an opening (411) defined on the rear side (113) and an inner end face (412) corresponding to the opening (411), the T-shaped trench (42) is indented on the inner end face (412) and the top side (116), and the handle (441) is exposed from the opening (411).

6. The nebulizer of claim 4, wherein a bottom and an outer periphery of the spraying seat (21) are disposed with a recess (211), the recess (211) comprises an inner bottom (212) and a U-shaped inner sidewall (213) disposed on a side of the inner bottom (212), the engaging trench (43) is indented on the inner bottom (212), and the two resistant bumps (421) abut against a bottom edge of the rod portion (4422) when the engaging block (443) is embedded in the engaging trench (43).

7. The nebulizer of claim 6, wherein the rails (32) are two pairs in number, two sides of the U-shaped inner sidewall (213) are disposed with two rail grooves (214) along the radial direction (d2), and each pair of rails (32) is disposed on an upper side and a lower side of each rail groove (214) correspondingly.

8. The nebulizer of claim 6, wherein the block (111) comprises a front side (112), the main body (1) comprises a power assembly (12) received in the body (11) and a first conductive member (13) installed on the front side (112) and electrically connected to the power assembly (12).

9. The nebulizer of claim 8, wherein an outside of the spraying seat (21) is extended with a nozzle (217) and an inside of the spraying seat (21) is disposed with an agent chamber (218) communicating with the nozzle (217), and the spraying head (2) further comprises a nebulizing sheet (24) installed at a junction between the nozzle (217) and the agent chamber (218).

10. The nebulizer of claim 9, wherein the spraying head (2) further comprises a second conductive member (22) and a no-water detection probe (23), the second conductive member (22) is installed on the U-shaped inner sidewall (213) and abuts against the first conductive member (13), an end of the no-water detection probe (23) stretches in the agent chamber (218) and is arranged corresponding to the nebulizing sheet (24), and the nebulizing sheet (24) is electrically connected with the second conductive member (22).

## Patentansprüche

1. Zerstäuber, umfassend:
einen Hauptkörper (1), der einen Körper (11) umfasst, wobei der Körper (11) eine axiale Richtung (d1) und eine radiale Richtung (d2) definiert;
einen Sprühkopf (2), der einen Sprühsitz (21) umfasst, der entlang der radialen Richtung (d2) an den Körper (11) montiert oder von diesem demontiert wird; und
eine Eingriffsstruktur (4), umfassend eine Kerbe (41), die an einem von dem Körper (11) und dem Sprühsitz (21) angeordnet ist, einen T-förmigen Graben (42), der an einer Innenwand der Kerbe (41) entlang der axialen Richtung (d1) angeordnet ist, einen Eingriffsgraben (43), der an einem anderen von dem Körper (11) und dem Sprühsitz (21) angeordnet ist und entsprechend der Kerbe (41) angeordnet ist, und ein bewegliches Element (44), das in der Kerbe (41) aufgenommen ist, wobei eine Seite des beweglichen Elements (44) mit einem Griff (441) verlängert ist, der von der Kerbe (41) freiliegt, eine andere Seite des beweglichen Elements (44) mit einem T-förmigen Block (442) verlängert ist, der gleitend in dem T-förmigen Graben (42) aufgenommen ist, und noch eine andere Seite des beweglichen Elements (44) mit einem Eingriffsblock (443) hervorsteht, der in dem Eingriffsgraben (43) eingebettet oder von diesem entlang der axialen Richtung getrennt ist.

2. Zerstäuber nach Anspruch 1, wobei der T-förmige Block (442) einen Kopfteil (4421) und einen Stabteil (4422) umfasst, der zwischen dem Kopfteil (4421) und dem beweglichen Element (44) verbunden ist, und eine Innenwand des T-förmigen Grabens (42) mit zwei widerstandsfähigen Höckern (421) verlängert ist, die mit dem Stabteil (4422) reiben.

3. Zerstäuber nach Anspruch 2, der ferner eine Gleitschienenstruktur (3) umfasst, wobei die Gleitschienenstruktur (3) mindestens einen Gleiter (31), der entweder am Körper (11) oder am Sprühsitz (21) angeordnet ist, und mindestens ein Paar Schienen (32) umfasst, die am anderen Körper (11) oder am Sprühsitz (21) angeordnet sind, das mindestens eine Paar von Schienen (32) entlang der radialen Richtung (d2) und in einer Oben-Unten-Konfiguration angeordnet ist, und der mindestens eine Schieber (31) zwischen dem mindestens einen Paar von Schienen (32) verschiebbar und arretierbar ist.

4. Zerstäuber nach Anspruch 3, wobei eine Oberseite des Körpers (11) mit einem Block (111) hervorsteht, der Block (111) eine Rückseite (113), eine linke Seite (114), eine rechte Seite (115) und eine Oberseite (116) umfasst, der Schieber (31) zwei an der Zahl ist, die beiden Schieber (31) von der linken Seite (114) und der rechten Seite (115) hervorstehen und die Kerbe (41) auf der Rückseite (113) und der Oberseite (116) eingekerbt ist.

5. Zerstäuber nach Anspruch 4, wobei die Kerbe (41) eine an der Rückseite (113) definierte Öffnung (411) und eine der Öffnung (411) entsprechende innere Endfläche (412) aufweist, der T-förmige Graben (42) an der inneren Endfläche (412) und der Oberseite (116) eingekerbt ist und der Griff (441) aus der Öffnung (411) herausragt.

6. Zerstäuber nach Anspruch 4, wobei ein Boden und ein äußerer Umfang des Sprühsitzes (21) mit einer Aussparung (211) angeordnet sind, wobei die Aussparung (211) einen inneren Boden (212) und eine U-förmige innere Seitenwand (213) umfasst, die an einer Seite des inneren Bodens (212) angeordnet ist, der Eingriffsgraben (43) am inneren Boden (212) eingekerbt ist, und die beiden widerstandsfähigen Höcker (421) an einer Unterkante des Stangenabschnitts (4422) anliegen, wenn der Eingriffsblock (443) in den Eingriffsgraben (43) eingebettet ist.

7. Zerstäuber nach Anspruch 6, wobei die Schienen (32) aus zwei Paaren bestehen, zwei Seiten der U-förmigen inneren Seitenwand (213) mit zwei Schienennuten (214) entlang der radialen Richtung (d2) angeordnet sind und jedes Paar von Schienen (32) auf einer oberen Seite und einer unteren Seite jeder Schienennut (214) entsprechend angeordnet ist.

8. Zerstäuber nach Anspruch 6, wobei der Block (111) eine Vorderseite (112) aufweist, der Hauptkörper (1) eine in dem Körper (11) aufgenommene Leistungsbaugruppe (12) und ein erstes leitendes Element (13) aufweist, das an der Vorderseite (112) angebracht und mit der Leistungsbaugruppe (12) elektrisch verbunden ist.

9. Zerstäuber nach Anspruch 8, wobei eine Außenseite des Sprühsitzes (21) mit einer Düse (217) verlängert ist und eine Innenseite des Sprühsitzes (21) mit einer Wirkstoffkammer (218) angeordnet ist, die mit der Düse (217) in Verbindung steht, und der Sprühkopf (2) ferner ein Zerstäubungsblatt (24) umfasst, das an einer Verbindung zwischen der Düse (217) und der Wirkstoffkammer (218) installiert ist.

10. Zerstäuber nach Anspruch 9, wobei der Sprühkopf (2) ferner ein zweites leitendes Element (22) und eine Wasserfreiheitserkennungssonde (23) umfasst, wobei das zweite leitende Element (22) an der U-förmigen inneren Seitenwand (213) installiert ist und an dem ersten leitenden Element (13) anliegt, ein Ende der wasserfreien Erfassungssonde (23) sich in die Wirkstoffkammer (218) erstreckt und entsprechend der Vernebelungsfolie (24) angeordnet ist, und die Vernebelungsfolie (24) elektrisch mit dem zweiten leitfähigen Element (22) verbunden ist.

## Revendications

1. Un atomiseur, comprenant:
un corps principal (1) comprenant un corps (11), le corps (11) définissant une direction axiale (d1) et une direction radiale (d2) ;
une tête de pulvérisation (2) comprenant un siège de pulvérisation (21) qui est monté sur le corps (11) ou démonté de celui-ci le long de la direction radiale (d2) ; et
une structure d'engagement (4) comprenant une encoche (41) disposée sur l'un du corps (11) et du siège de pulvérisation (21), une tranchée en forme de T (42) disposée sur une paroi intérieure de l'encoche (41) le long de la direction axiale (d1), une tranchée d'engagement (43) située sur l'autre du corps (11) et du siège de pulvérisation (21) et disposée en correspondance avec l'encoche (41), et un élément mobile (44) reçu dans l'encoche (41), dans lequel un côté de l'élément mobile (44) est prolongé par une poignée (441) exposée à partir de l'encoche (41), un autre côté de l'élément mobile (44) est prolongé par un bloc en forme de T (442) reçu de manière coulissante dans la tranchée en forme de T (42), et encore un autre côté de l'élément mobile (44) fait saillie avec un bloc d'engagement (443) encastré dans ou séparé de la tranchée d'engagement (43) le long de la direction axiale.

2. L'atomiseur selon la revendication 1, dans lequel le bloc en forme de T (442) comprend une partie de tête (4421) et une partie de tige (4422) reliée entre la partie de tête (4421) et l'élément mobile (44), et une paroi interne de la tranchée en forme de T (42) est prolongée par deux bosses résistantes (421) qui frottent avec la partie de tige (4422).

3. L'atomiseur selon la revendication 2, comprenant en outre une structure de glissière (3), ladite structure de glissière (3) comprenant au moins un coulisseau (31) disposé soit sur le corps (11) soit sur le siège de pulvérisation (21), et au moins une paire de rails (32), disposés sur l'autre corps (11) ou sur le siège de pulvérisation (21), ladite au moins une paire de rails (32) étant disposée le long de la direction radiale (d2) et dans une configuration haut-bas, et ledit au moins un coulisseau (31) pouvant être déplacé et verrouillé entre ladite au moins une paire de rails (32).

4. L'atomiseur selon la revendication 3, dans lequel une face supérieure du corps (11) fait saillie avec un bloc (111), le bloc (111) comprend une face arrière (113), un côté gauche (114), un côté droit (115) et une face supérieure (116), le curseur (31) est au nombre de deux, les deux curseurs (31) font saillie du côté gauche (114) et du côté droit (115), et l'encoche (41) est entaillée sur la face arrière (113) et la face supérieure (116).

5. L'atomiseur selon la revendication 4, dans lequel l'encoche (41) présente une ouverture (411) définie sur la face arrière (113) et une surface d'extrémité intérieure (412) correspondant à l'ouverture (411), la tranchée en forme de T (42) est encochée sur la surface d'extrémité intérieure (412) et la face supérieure (116), et la poignée (441) fait saillie de l'ouverture (411).

6. L'atomiseur selon la revendication 4, dans lequel un fond et une périphérie extérieure du siège de pulvérisation (21) sont agencés avec un évidement (211), l'évidement (211) comprenant un fond intérieur (212) et une paroi latérale intérieure en forme de U (213) agencée sur un côté du fond intérieur (212), la tranchée d'engagement (43) est entaillée au niveau du fond interne (212), et les deux bosses résistantes (421) sont en butée contre un bord inférieur de la section de tige (4422) lorsque le bloc d'engagement (443) est encastré dans la tranchée d'engagement (43).

7. L'atomiseur selon la revendication 6, dans lequel les rails (32) sont constitués de deux paires, deux côtés de la paroi latérale interne en forme de U (213) sont disposés avec deux rainures de rail (214) le long de la direction radiale (d2), et chaque paire de rails (32) est disposée sur un côté supérieur et un côté inférieur de chaque rainure de rail (214) respectivement.

8. L'atomiseur selon la revendication 6, dans lequel le bloc (111) comprend une face avant (112), le corps principal (1) comprend un ensemble de puissance (12) logé dans le corps (11) et un premier élément conducteur (13) monté sur la face avant (112) et relié électriquement à l'ensemble de puissance (12).

9. L'atomiseur selon la revendication 8, dans lequel un côté extérieur du siège de pulvérisation (21) est prolongé par une buse (217) et un côté intérieur du siège de pulvérisation (21) est agencé avec une chambre d'agent actif (218) communiquant avec la buse (217), et la tête de pulvérisation (2) comprend en outre une lame d'atomisation (24) installée à une jonction entre la buse (217) et la chambre d'agent actif (218).

10. L'atomiseur selon la revendication 9, dans lequel la tête de pulvérisation (2) comprend en outre un deuxième élément conducteur (22) et une sonde de détection d'absence d'eau (23), le deuxième élément conducteur (22) étant installé sur la paroi latérale interne en forme de U (213) et étant en butée contre le premier élément conducteur (13), une extrémité de la sonde de détection anhydre (23) s'étend dans la chambre d'agent (218) et est disposée en correspondance avec la feuille de nébulisation (24), et la feuille de nébulisation (24) est connectée électriquement au deuxième élément conducteur (22).
